**Europäisches Patentamt**

(19) **European Patent Office**

**Office européen des brevets**

(11) Numéro de publication: **0 120 790**
**B1**

(12) **FASCICULE DE BREVET EUROPEEN**

(45) Date de publication du fascicule du brevet:
03.06.87

(21) Numéro de dépôt: **84400600.7**

(22) Date de dépôt: **23.03.84**

(51) Int. Cl.⁴: **A 41 B 13/02**, A 61 F 13/16

(54) Couche à jeter, notamment pour adulte incontinent.

(30) Priorité: **29.03.83 FR 8305123**

(43) Date de publication de la demande:
**03.10.84 Bulletin 84/40**

(45) Mention de la délivrance du brevet:
**03.06.87 Bulletin 87/23**

(84) Etats contractants désignés:
**BE DE FR GB LU NL**

(56) Documents cités:
**FR - A - 2 425 205**
**GB - A - 2 001 236**
**GB - A - 2 972 491**
**US - A - 1 733 429**
**US - A - 3 126 600**
**US - A - 4 158 906**
**US - A - 4 300 562**

(73) Titulaire: **BEGHIN-SAY SOCIETE ANONYME,**
**F-59239 Thumeries (FR)**

(72) Inventeur: **Mitrani, Sem, 16 rue des Bergeronnettes,**
**F-91130 Ris Orangis (FR)**

(74) Mandataire: **Quéré, Jean Pierre, BEGHIN-SAY Service**
**Propriété Industrielle 54, Avenue Hoche, F-75008 Paris**
**(FR)**

ACTORUM AG

## Description

La présente invention a pour objet une couche à jeter, notamment pour adulte incontinent, constituée d'un tampon plat absorbant, de forme rectangulaire, ledit tampon étant recouvert sur sa face externe d'une feuille imperméable aux liquides et sur sa face interne en contact avec la peau de l'utilisateur d'une feuille permeable aux liquides en non tissé, ladite couche ayant une zone arrière et une zone avant.

Plusieurs couches pour adulte incontinent ont déjà été proposées. Parmi celles-ci, on peut citer une couche munie de deux bandes adhésives situées sur chacun des bords longitudinaux arrières de celle-ci. En position, chacune des bandes adhésives est fixée sur les bords longitudinaux avants correspondants.

Ce type d'attache présente, toutefois, certains inconvénients. En raison de la position des attaches, l'utilisateur est obligé de se mettre dos au mur ou de s'asseoir pour positionner la couche.

Cette disposition nécessite, en outre, en pratique, que la change se recouvre sur les côtés.

Le modèle de couche proposé dans cette invention a pour but de pallier les deux inconvénients décrits ci-dessus, en outre permet d'apporter les avantages suivants:
* une meilleure étanchéité,
* la fabrication d'un modèle unique convenant à toutes les tailles.

L'invention est caractérisée en ce que les dispositifs d'attaches sont formés de deux ceintures élastiques et en ce qu'avant utilisation une des deux ceintures élastiques, constituée d'une bande élastique munie à chacune de ses extrémités d'une pince, est fixée au niveau de la zone arrière sur l'un des bords longitudinaux de façon à ce que la pince utilisée enserre le bourrelet, et l'autre ceinture, similaire à la première, est fixée sur le même bord longitudinal au niveau de la zone avant de façon à ce que la pince utilisée de cette seconde ceinture enserre également le bourrelet.

Un tampon absorbant consiste généralement en mousse de cellulose dans laquelle peuvent être incorporés des matériaux hydrocolloïdaux absorbant plusieurs fois leur poids en liquide.

La feuille imperméable aux liquides est généralement en polyéthylène.

La couche peut être quasi rectangulaire: il n'y a donc pas besoin de prévoir des extensions latérales dans les zones arrière et avant. Elle peut être plane d'un seul niveau ou au contraire repliée symétriquement par rapport à son axe longitudinal pour obtenir plusieurs épaisseurs superposées comme décrit par exemple dans le brevet FR 2 181 792.

La zone arrière d'une couche est la zone qui, lorsque la couche est positionnée, est située au niveau de la taille dans le dos de l'utilisateur. La zone avant est la zone opposée.

Le bourrelet sera, par exemple, réalisé au moyen d'un cordon inséré entre une feuille et le tampon, ou bien, lorsque les feuilles s'étendent latéralement au delà des bords du tampon le cordon pourra être pris en sandwich entre les deux feuilles.

De préférence, ce cordon est élastique et a été fixé à l'état étiré sur toute la longueur du bord longitudinal, l'élasticité des bandes élastiques est ainsi combinée à l'élasticité du cordon, ce qui assure une meilleure étanchéité.

On connaît le brevet FR 2 425 205 qui décrit une couche-culotte pourvue de liens souples guidés, chacun, dans une gaine ménagée le long des bords longitudinaux du matelas. Ces liens permettent, après la mise en place de la couche-culotte sur le bébé de façon traditionnelle, de fermer plus ou moins, à volonté les passages des jambes. On note, toutefois, que le dispositif d'attache d'une part est traditionnel, donc du type à bande adhésive, d'autre part ne coopère pas avec le bourrelet constitué par la gaine.

L'invention sera mieux comprise à l'aide de la description détaillée qui va suivre d'un exemple préféré de réalisation et des dessins annexés sur lesquels:

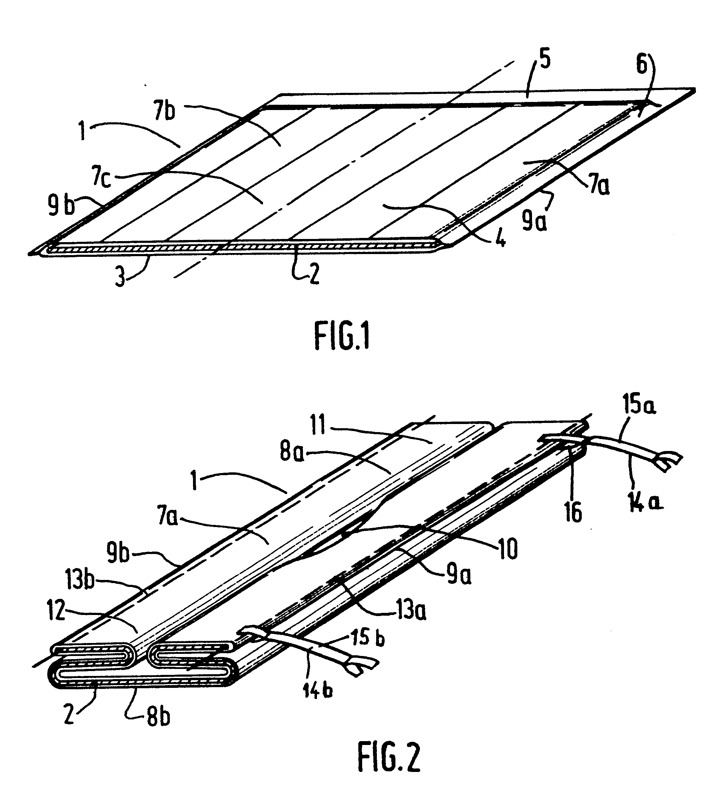

— La figure 1 est une vue en perspective d'une couche mise à plat avec une coupe en révélant les différents constituants,
— La figure 2 est une vue en perspective d'une couche prête à être utilisée avec les attaches élastiques fixées,
— Les figures 3, 4, 5 montrent la manière de positionner la couche pour l'utilisateur.

Une couche rectangulaire 1 est constituée d'un tampon absorbant 2 en mousse de cellulose recouvert sur une face d'une feuille imperméable 3 en polyéthylène et, sur l'autre face, d'une feuille permeable 4 en non-tissé. La surface du tampon absorbant est plus petite que celle des feuilles 3 et 4 ce qui permet de fermer la poche et de former des bandes latérales 6 et longitudinales 5.

Cette couche est pourvue de quatre lignes de pliage longitudinales disposées symétriquement deux à deux et parallèlement à son axe longitudinal.

Ces lignes de pliage délimitent cinq bandes:
— deux bandes externes 7a,
— deux bandes 7b adjacentes aux bandes externes situées de part et d'autre d'une
— bande médiane 7c dont la surface est sensiblement égale au double de la surface de chacune des bandes 7a et 7b.

En position d'utilisation, les bandes 7b adjacentes sont repliées de manière à ce qu'elles recouvrent environ une demi-surface du non tissé 4 de la bande médiane 7c, tandis que par retour les bandes externes 7a sont repliées de manière à ce que la face en polyéthylène de ces bandes recouvre la face en polyéthylène des bandes 7b adjacentes aux bandes externes.

Ainsi, les surfaces en non tissé des bandes externes 7a constituent, éventuellement avec une partie de la bande médiane 7c, la face interne 8a de la couche destinée à venir en contact avec la peau de l'utilisateur et la surface en polyéthylène de la bande médiane 7c constitue la face externe 8b de la couche. Les bords extérieurs des bandes externes 7a forment les bords longitudinaux 9a et 9b de la couche. Les surface en non-tissé des bandes adjacentes 7b peuvent être rendues solidaires de la bande médiane 7c au moins au niveau de la zone centrale 10.

A proximité des bords longitudinaux 9a et 9b et parallèlement à eux des cordons élastiques 13a et 13b à l'état étiré sont insérés entre les feuilles 3 et 4 au niveau des bandes latérales 6.

Ces cordons élastiques 13a et 13b jouent deux rôles:

– faciliter le maintien des moyens de fixation qui vont être décrits ci-après,

– assurer une meilleure étanchéité en «prolongeant» l'élasticité des bandes élastiques.

Les ceintures élastiques 14a et 14b sont constituées d'une bande élastique 15a, 15b terminée à chaque extrémité par des pinces 16 en forme de mâchoire. La longeur de ces ceintures peut être avantageusement réglable.

Une des pinces 16 d'une première ceinture élastique 14a est fixée transversalement par rapport à l'axe longitudinal, au niveau du bord longitudinal 9a dans la zone arrière 11 de manière à enserrer le cordon élastique 13a et être située au niveau de la taille de l'utilisateur. Une des pinces 16 d'une deuxième ceinture élastique 14b est fixée transversalement au niveau du même bord longitudinal 9a dans la zone avant 12 de manière à enserrer le cordon élastique 13a et être située au niveau de la taille.

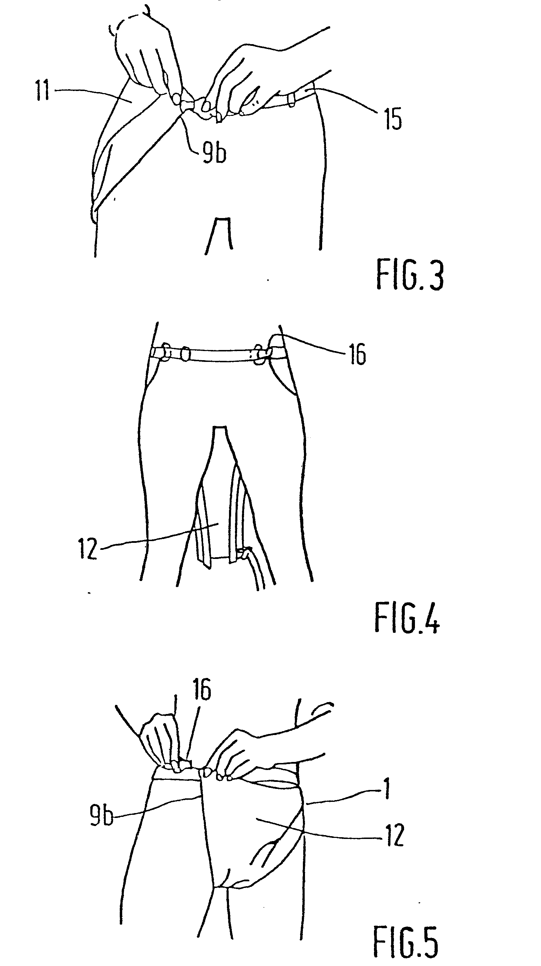

Selon les figures 3, 4, 5 la face interne 8a de la zone arrière 11 posée contre la hanche, le bord longitudinal 9a, portant les ceintures élastiques 14a, 14b dirigé vers le dos, l'utilisateur ramène la pince 16 libre de l'attache élastique 14a, située au niveau de la zone arrière 11, devant lui en la faisant passer derrière son dos et la fixe à l'autre bord longitudinal 9b de la zone arrière 11 puis déplace la zone arrière 11 de la couche vers le dos. La zone avant 12 de la couche est ensuite remontée entre les jambes et la pince libre de l'autre ceinture élastique 14b est ramenée en la faisant passer derrière le dos de l'utilisateur, vers l'avant, de manière à la fixer à l'autre bord longitudinal 9b de la zone avant 12.

## Revendications

1. Couche à jeter, notamment pour adulte incontinent, constituée d'un tampon plat (2) absorbant, de forme rectangulaire, pourvu de dispositifs d'attaches, ledit tampon (2) étant recouvert sur sa face externe d'une feuille imperméable (3) et, sur sa face interne en contact avec la peau de l'utilisateur, d'une feuille perméable (4) en non-tissé, des bourrelets (13a, 13b) étant présents à proximité de chacun des bords longitudinaux (9a, 9b), caractérisée en ce que les dispositifs d'attaches sont formés de deux ceintures élastiques (14a, 14b) et en ce qu'avant utilisation une des deux ceintures élastiques, (14a), constituée d'une bande élastique munie à chacune de ses extrémités d'une pince (16), est fixée au niveau de la zone arrière (11) sur l'un des bords longitudinaux (9a) de façon à ce que la pince utilisée enserre le bourrelet (13a), et l'autre ceinture (14b), similaire à la première, est fixée sur le même bord longitudinal (9a) au niveau de la zone avant (12) de façon à ce que la pince utilisée de la seconde ceinture enserre également le bourrelet (13a).

2. Couche à jeter selon la revendication 1, caractérisée en ce que les bourrelets sont des cordons (13a, 13b) enserrés entre la feuille perméable (4) et la feuille imperméable (3).

3. Couche à jeter selon l'une des revendications 1 ou 2, caractérisée en ce que les bourrelets (13a, 13b) sont élastiques et s'étendent sur toute la longueur de leur bord longitudinal respectif (9a, 9b).

4. Couche à jeter selon l'une des revendications précédentes caractérisée en ce que les bandes élastiques (15a, 15b) sont fixées transversalement par rapport à l'axe longitudinal de la couche.

## Patentansprüche

1. Wegwerfwindel, insbesondere für inkontinenzkranke Erwachsene, bestehend aus einem absorbierenden flachen Tampon (2) von rechteckiger Form, der mit Befestigungseinrichtungen versehen ist, auf seiner Aussenseite mit einer undurchlässigen Folie (3) und auf seiner mit der Haut des Benutzers in Berührung befindlichen Innenseite mit einer durchlässigen Folie (4) aus Nichtgewebe bedeckt ist, wobei Wülste (13a, 13b) in der Nähe jeder Längskante (9a, 9b) vorhanden sind, dadurch gekennzeichnet, dass die Befestigungseinrichtungen aus zwei elastischen Bändern (14a, 14b) gebildet sind und vor Benutzung eines der zwei elastischen Bänder, und zwar (14a), welches aus einem an jedem seiner Enden mit einer Klemme (16) versehenen Gummiband besteht, in der Ebene der hinteren Zone (11) auf einer der Längskanten (9a) derart befestigt wird, dass die verwendete Klemme den Wulst (13a) einklemmt, und das andere Band (14b) ähnlich dem ersten auf derselben Längskante (9a) in der Ebene der vorderen Zone (12) derart befestigt wird, dass die verwendete Klemme des zweiten Bandes ebenso den Wulst (13a) einklemmt.

2. Wegwerfwindel nach Anspruch 1, dadurch gekennzeichnet, dass die Wülste zwischen der durchlässigen Folie (4) und der undurchlässigen Folie (3) eingeklemmte Schnüre (13a, 13b) sind.

3. Wegwerfwindel nach einem der Ansprüche 1 oder 2, dadurch gekennzeichnet, dass die Wülste (13a, 13b) elastisch sind und sich über die gesamte Länge ihrer jeweiligen Längskante (9a, 9b) erstrecken.

4. Wegwerfwindel nach einem der vorhergehenden Ansprüche, dadurch gekennzeichnet, dass die Gummibänder (15a, 15b) quer zur Längsachse der Windel befestigt sind.

## Claims

1. Disposable nappy, particularly for an incontinent adult, consisting of a flat absorbent pad (2) of rectangular shape provided with fastening devices, the said pad (2) being covered on its outer face with an impervious sheet (3) and, on its inner face in contact with the user's skin, with a permeable nonwoven sheet (4) ridges (13a, 13b) being present in the vicinity of each of the lengthwise edges (9a, 9b) characterized in that the fastening devices consist of two elastic belts (14a, 14b) and in that before use one of the two elastic belts (14a), consisting of an elastic strip provided with a clamp (16) at each of its ends, is attached onto one of the lengthwise edges (9a) in the rear region (11) so that the clamp employed grips the ridge (13a), and the other belt (14b), similar to the first, is attached onto the same lengthwise edge (9a) in the front region (12) so that the employed clamp of the second belt also grips the ridge.

2. Disposable nappy according to claim 1, characterized in that the ridges are cords (13a, 13b) gripped between the permeable sheet (4) and the impervious sheet (3).

3. Disposable nappy according to either of claims 1 and 2, characterized in that the ridges (13a, 13b) are elastic and extend over the whole length of their respective lengthwise edges (9a, 9b).

4. Disposable nappy according to one of the preceding claims, characterized in that the elastic strips (15a, 15b) are attached transversely in relation to the lengthwise axis of the nappy.

FIG.1

FIG.2

FIG.3

FIG.4

FIG.5

7